# EUROPÄISCHE PATENTSCHRIFT

(11) **EP 0 945 146 B1**
(45) Veröffentlichungstag und Bekanntmachung des Hinweises auf die Patenterteilung: **14.05.2003**
(21) Anmeldenummer: 99105434.7
(22) Anmeldetag: 17.03.1999
(51) Int. Cl.: A61L 27/46, C07K 1/00, C08L 89/06

(54) **Verfahren zur Herstellung von mineralisierten Kollagenfibrillen und deren Verwendung als Knochenersatzwerkstoff**
Method for making mineralized collagen fibrils and their use as bone replacement material
Procédé de fabrication de fibrilles de collagène minéralisées et leur utilisation comme matériaux de remplacement osseux

(30) Priorität: 24.03.1998 DE 19812714
(43) Veröffentlichungstag der Anmeldung: 29.09.1999
(73) Patentinhaber: MERCK PATENT GmbH, 64293 Darmstadt (DE)
(72) Erfinder: Weis, Karl, Dr., 79263 Simonswald (DE); Pompe, Wolfgang, Prof., 01737 Kurort Harta (DE); Bradt, Jens, 01099 Dresden (DE)

(56) Entgegenhaltungen:
- GB-A- 1 068 587
- US-A- 5 320 844
- US-A- 5 532 217
- WEINER S., TRAUB W.: "The mineralized Collagen Fibril : A building block for many vertebrate skeletal tissue" INTERNATIONAL CONGRESS SERIES., Bd. 1002, 1992, Seiten 93-102, XP000089821

## Beschreibung

Die Erfindung betrifft ein Verfahren zur Herstellung von mineralisierten Kollagenfibrillen, wobei die Fibrillenbildung und Mineralisierung in einem Prozeßschritt ablaufen. Ein weiterer Gegenstand der Erfindung ist ein Verfahren zur Herstellung eines Kollagen/Calciumphosphatkomposits ausgehend von mineralisierten Kollagenfibrillen, die in einen Calciumphosphatzement eingebettet werden. Dieser dient dann als Knochenersatzmaterial.

Kollagenes Gewebe besitzt als Biomaterial gegenüber anorganischem Material viele Vorteile. Kollagen ist ein wichtiges Protein bei Tieren und macht etwa 30 % der Proteine der Wirbeltiere aus. Es besitzt die Fähigkeit, in vitro in Fibrillen zu assemblieren, wobei diese in verschiedenen Modifikationen hergestellt werden können. Im menschlichen Knochen stellt Kollagen als Strukturmaterial die organische Matrix dar, in der Mineralien bestehend aus Calcium-, Phosphat-, Hydroxyl-, Fluorid- und Carbonationen etc. eingelagert werden.
Aus der Ähnlichkeit zu menschlichem Gewebe ergibt sich ein wichtiger Vorteil gegenüber alternativen Materialien. Dieser Vorteil wird ausgenutzt um Prothesen und Biomaterialien herzustellen.
Aus dem Stand der Technik sind zahlreiche Dokumente bekannt, die sich mit der Mineralisierung von Kollagen beschäftigen.

Die GB-1068 587 (Baxendale et. al, 1963) offenbart lösliches Kollagen (aus Rattenschwänzen), welches rekonstituiert und durch Immersion in einer übersättigten Calciumphosphatlösung mineralisiert wird. Die Assemblierung in Gegenwart von ATP oder Chondroitinsulfat führt zu sog. SLS bzw. FLS (vgl. G. Reich "Kollagen", Theodor Steinkopff, Dresden (1966) S. 134 ff.) und nicht wie irrtümlich angenommen zu Kollagenfibrillen mit 64 nm-Periode.

Die US- 5320 844 (Lui, 1992) beschreibt quervemetztes Kollagen, welches in einer sauren Lösung dispergiert und durch Zugabe einer Calcium- und Phosphatlösung mit neutralem pH mineralisiert wird. Entweder werden beide Lösungen gleichzeitig zugegeben, oder eine der beiden Lösungen wird vorher mit dem Kollagen vermischt.

Die US-5455 231, US-5231 169 und WO-93/12736 (Constantz et al.) beschreiben ein quervemetztes Kollagen, welches in basischer Lösung solubilisiert und anschließend eine Calcium- und eine Phosphatlösung über mehrere Stunden zugetropft wird. Ein Assemblierungschritt findet hier nicht statt.

Die US-5532 217 (Silver et al., 1995) offenbart Kollagenfasem, die aus einer sauren Kollagenlösung entstehen, welche in einem Neutralisierungspuffer extrudiert wird. Die Fasern, die einen Durchmesser von 20 bis 500 µm besitzen, werden anschließend mineralisiert, z.B. in einer Doppeldiffusionskammer.

Die vorliegende Erfindung hat sich die Aufgabe gestellt, ein Verfahren zur Herstellung von mineralisierten Kollagenfibrillen bereitzustellen, womit ein homogen mineralisiertes Kollagengel entsteht. Dabei soll nur natives, also nicht rekombinantes Kollagen verwendet werden. Eine weitere Aufgabe bestand darin, ein Verfahren zur Herstellung eines Kollagen/Calciumphosphatkomposits unter Verwendung der mineralisierten Fibrillen zur Verfügung zu stellen, wodurch ein besserer Verbund zwischen Kollagen und Calciumphosphatzement gewährleistet wird.

Die erste Aufgabe konnte überraschend einfach durch ein Verfahren zur Herstellung von mineralisierten Kollagenfibrillen gelöst werden, indem Fibrillenbildung und Mineralisierung in einem Prozeßschritt ablaufen.
Die zweite Aufgabe konnte durch ein Verfahren zur Herstellung eines Kollagen/Calciumphosphatkomposits gelöst werden, indem die mineralisierten Kollagenfibrillen in einem Calciumphosphatzement bei einem bestimmten Gewichtsverhältnis eingebettet werden.

Die Herstellung der mineralisierten Kollagenfibrillen erfolgt aus löslichem Kollagen. Die Verwendung von löslichem Kollagen beinhaltet die Möglichkeit einer höheren Aufreinigung gegenüber der Verwendung von nicht löslichem Kollagen, was das Risiko von Krankheitsübertragungen und Gewebeunverträglichkeit verringert. Die Möglichkeit, aus löslichem Kollagen Fibrillen zu rekonstituieren, ist seit langem bekannt (A. Veis, K. Payne "Collagen Fibrillogenesis" aus Collagen" ed. M. E. Nimni Vol.1, Biochemistry, CRC Boca Raton 1988).

Nicht quervemetztes Kollagen ist bei niedrigem pH (pH ≈ 2) löslich und assembliert sich bei neutralem pH zu Fibrillen. Die Rekonstitution erfolgt durch Mischen einer sauren Kollagenlösung mit einer neutralen Pufferlösung.
Als saure Kollagenlösung kommen alle Kollagenlösungen in Betracht, die gelöstes Calciumsalz bei einem sauren pH-Wert, vorzugsweise pH =2, enthalten.
Als Pufferlösungen kommen alle neutralen Pufferlösungen in Betracht.

Die Mineralisierung der Kollagenfibrillen erfolgt durch Ausfällen von Calciumphosphat aus einer übersättigten Calciumphosphatlösung. Die Übersättigung wird erzeugt durch Mischen einer Calcium- und einer Phosphatkomponente. Dadurch, daß die Calcium- und die Phospatkomponente zeitgleich mit der Kollagenlösung und der Pufferlösung gemischt werden, werden beide Prozesse gleichzeitig initiiert. Durch geeignete Wahl der Parameter muß eine geeignete Abfolge der Prozesse erreicht werden, d.h. die Fibrillenbildung muß vor der Mineralisierung beginnen, da die Kollagenfibrillen als Template (Substrat) für die Mineralisierung dienen sollen. Zugleich muß die Ausbildung einer dichten Kollagenstruktur soweit begrenzt werden, daß in einer praktisch sinnvollen Zeit noch ein hinreichendes Eindiffundieren von Calcium- und Phosphationen in die Kollagenfibrillen ermöglicht wird. Die gewünschte Abfolge der Prozesse wird nur in einem engen Parameterbereich erreicht.
Die erhaltenen Kollagenfibrillen besitzen im Gegensatz zu Kollagenfasem lediglich einen Durchmesser von 20-500 nm.
Bei der Mineralisierung können den erfindungsgemäßen Kollagenfibrillen gewünschtenfalls Polyaspartat, Polyglutamat, Polyphosphoserin, andere Polycarboxylate oder nichtkollagene Proteine oder Phosphoproteine zugesetzt werden, um die Mineralisierung zu verbessern und die Kinetik des Mineralisationsprozesses zu beeinflussen. Weiterhin ist der Zusatz von Knochenwachstumsfaktoren denkbar, die die Knochenneubildung anregen.

Es ist erfindungsgemäß weiterhin möglich, die mineralisierten Kollagenfibrillen in einem zweiten Verfahrensschritt in einen Calciumphosphatzement einzubetten. Der Calciumphosphatzement wird hergestellt aus Tetracalciumphosphat, Calciumhydrogenphosphat, Calciumhydroxylapatit, Calciumdihydrogenphosphat, Calciumcarbonat, Natriumphosphaten und/oder Gemischen davon, wobei das Gewichtsverhältnis des entstehenden Komposits Kollagen Calciumphosphat (CaP) 1:200 bis 1:1 beträgt.
Die Einzelkomponenten des Calciumphosphatzementes werden im festen Zustand auf eine geeignete Teilchengröße zerkleinert und durch Vermischen mit einer flüssigen Komponente zu einer plastischen Masse angerührt. Die Aushärtung der so gewonnenen Zementmischungen verläuft in zwei Schritten:
- Zunächst über einen definierten Zeitraum an Luft bei Raumtemperatur
- Anschließend über einen ebenfalls definierten Zeitraum in einer wäßrigen Phase, die Phosphat oder andere mineralische Bestandteile enthalten kann.

Die Einbettung der mineralisierten Kollagenfibrillen erfolgt durch Einmischen in den Zementprecursor. Der Vorteil der Einbettung mineralisierter Kollagenfibrillen besteht in der Vernetzung mit der Matrixphase (Calciumphosphatzement), was zu einer erhöhten Bruchzähigkeit führt.

Gewünschtenfalls können die o.g. nichtkollagenen Zusätze auch erst beim Einbetten des Knochenzementes zugegeben werden.

Die Einbettung der Kollagenfibrillen kann, je nach Anforderungsprofil, entweder orientiert oder nicht orientiert erfolgen. Dabei kann die Orientierung durch die Einbettung von Laminaten aus orientierten Fibrillen erreicht werden. Eine orientierte Einbettung ermöglicht einen höheren Kollagenanteil im Komposit. Außerdem kann auf diese Weise ein Material mit anisotropen mechanischen Eigenschaften, wie sie auch im Knochen vorliegen, erzeugt werden.

In den folgenden Beispielen sind Ausführungsformen der mineralisierten Kollagenfibrillen und des Kollagen/ Calciumphosphatkomposits näher erläutert.

### Herstellung von mineralisierten Kollagenfibrillen:

### Beispiel 1

Lösliches Kollagen I (aus Kalbshaut) wird in einer Konzentration von 1 mg/ml in 10 mM Salzsäure bei einer Temperatur von 4 °C aufgelöst. Die Lösung wird entweder am selben Tag verarbeitet oder tiefgefroren bei -80 °C gelagert.
Komponente 1 wird durch Mischen von 700 µl Kollagenlösung und 126 µl wäßriger Calciumchloridlösung (0.1 mol/l) bei 4 °C hergestellt.
Komponente 2 wird durch Mischen von 165 µl wäßriger Natriumchloridlösung (2 mol/l), 240 µl wäßriger Tris-(hydroxymethyl)-aminomethan-lösung (0.5 mol/l, pH 7.4), 32.4 µl wäßriger KH₂PO₄ /K₂HPO₄-Lösung (0.5 mol/l, pH 7.4) und 793 µl Wasser bei 4 °C hergestellt.
Durch Zugabe von 574 µl Komponente 2 zu Komponente 1 und rasche Temperaturerhöhung auf 30 °C werden Fibrillenlösung und Mineralisierung initiiert.
Das Gemisch wird bei 30 °C inkubiert. Zunächst bilden sich Kollagenfibrillen, während sich gleichzeitig eine amorphe Calciumphosphatphase bildet. Nach ca. 90 Minuten wandelt sich die amorphe Calciumphosphatphase durch einen Lösungs/Wiederausfällungsmechanismus in kristallinen Defektapatit um. Der neugebildete Defektapatit scheidet sich dabei bevorzugt auf den Kollagenfibrillen ab, wobei sich größere Cluster von Calciumphosphatkristallen bilden.
Das Produkt hat eine gelartige Konsistenz. Die anschließende Aufarbeitung erfolgt durch Zentrifugation des mineralisierten Kollagens und Dekantieren des Überstandes. Danach wird in dest. Wasser resuspendiert Dieser Zyklus wird mehrmals wiederholt, um Puffersalze auszuwaschen. Schließlich wird gefriergetrocknet.

### Beispiel 2:

Kollagenlösung und Komponente 1 wie in Beispiel 1. Komponente 2 wie in Beispiel 1 jedoch zusätzlich 37.5 µl wäßrige Natriumpoly-Laspartat-lösung (4 mg/ml) und nur 755 µl Wasser enthaltend. Die Präparation erfolgt wie in Beispiel 1.
Wie in Beispiel 1 bilden sich zunächst Kollagenfibrillen und amorphes Calciumphosphat. Die Umwandlung von amorphen Calciumphosphat zum Defektapatit erfolgt jedoch erst nach ca. 8 Stunden. In diesem Fall scheiden sich einzelne Calciumphosphatkristallite ausschließlich auf und in den Kollagenfibrillen ab. Die Verbindung von Calciumphosphatkristallen und Kollagen ist gegenüber Beispiel 1 eindeutig verbessert. Aufarbeitung analog zu Beispiel 1.

### Einbetten der mineralisierten Kollagenfibrillen in einen Calciumphosphatzement

### Beispiel 3

Zu einem Gemisch aus 500 mg Calciumdihydrogenphosphat und Calciumcarbonat wurden 5 mg mineralisierte Kollagenfibrillen zugegeben. Die Mischung wurde mit 230 ml Phosphatpuffer zu einer plastischen Masse angerührt, welche zunächst an Luft und anschließend in einem Phosphatpuffer aushärtete.

### Beispiel 4

Zu einem Gemisch aus 1 g Calciumdihydrogenphosphat und Calciumcarbonat wurden 150 mg Natriumphosphat und 288 mg mineralisierte Kollagenfibrillen hinzugegeben. Die Mischung wurde mit Wasser zu einer plastischen Masse angerührt, welche bei 100 % Luftfeuchtigkeit aushärtete.

## Patentansprüche

1. Verfahren zur Herstellung von mineralisierten Kollagenfibrillen, **dadurch gekennzeichnet, daß** Fibrillenbildung und Mineralisierung in einem Prozeßschritt ablaufen.

2. Verfahren gemäß Anspruch 1, **dadurch gekennzeichnet, daß** die Fibrillenbildung vor der Mineralisierung beginnt.

3. Verfahren nach den Ansprüchen 1 und/oder 2, **dadurch gekennzeichnet, daß** nicht quervernetztes Kollagen eingesetzt wird.

4. Verfahren nach den Ansprüchen 1 und/oder 3, bestehend aus den Reaktionsschritten:
- Mischen einer sauren Kollagenlösung, die gelöstes Calciumsalz enthält, mit einer Pufferlösung, die Phosphat und Natriumchlorid enthält,
- Assemblierung des Kollagens zu Fibrillen durch Inkubieren eines Precursors bei einer geeigneten Temperatur und Mineralisierung des Calciumphosphats.
- sowie übliche Reinigungsschritte.

5. Verfahren nach einem der Ansprüche 1 bis 4, **dadurch gekennzeichnet, daß** die Kollagenfibrillen einen Durchmesser von 20-500 nm besitzen.

6. Verfahren nach einem der Ansprüche 1 bis 5, dadurch ge**gekennzeichnet, daß** Polycarboxylate wie Polyaspartat, Polyglutamat, Polyphophoserin oder nichtkollagene Proteine oder Phosphoproteine zugesetzt werden.

7. Verfahren nach einem der Ansprüche 1 bis 6, **dadurch gekennzeichnet, daß** natives Kollagen eingesetzt wird.

8. Verfahren zur Herstellung eines Kollagen/Calciumphosphatkomposits ausgehend von mineralisierten Kollagenfibrillen hergestellt nach einem Verfahren gemäß den Ansprüchen 1 bis 7, **dadurch gekennzeichnet, daß** die mineralisierten Kollagenfibrillen in einem Calciumphosphatzement, bestehend aus Calciumphosphaten wie Tetracalciumphosphat, Calciumhydrogenphosphat, Calciumhydroxylapatit, Calciumdihydrogenphosphat und/oder Calciumcarbonat, Natriumphosphaten und /oder Gemischen davon, bei einem Gewichtsverhältnis Kollagen:CaP von 1:200 bis 1:1 eingebettet werden.

9. Verfahren nach Anspruch 8, **dadurch gekennzeichnet, daß** die Einbettung der mineralisierten Kollagenfibrillen nicht orientiert ist.

10. Verfahren nach Anspruch 8, **dadurch gekennzeichnet, daß** eine orientierte Einbettung der mineralisierten Kollagenfibrillen durch Laminate aus orientierten Fibrillen erfolgt.

11. Verwendung von Kollagenfibrillen hergestellt nach einem der Ansprüche 1 bis 7 zur Herstellung von Knochenersatzmaterial.

## Claims

1. Process for the production of mineralised collagen fibrils, **characterised in that** fibril formation and mineralisation take place in a single process step.

2. Process according to Claim 1, **characterised in that** the fibril formation begins before the mineralisation.

3. Process according to Claims 1 and/or 2, **characterised in that** uncrosslinked collagen is employed.

4. Process according to Claims 1 and/or 3, consisting of the reaction steps:
- mixing of an acidic collagen solution comprising dissolved calcium salt with a buffer solution comprising phosphate and sodium chloride,
- assembly of the collagen into fibrils by incubation of a precursor at a suitable temperature and mineralisation of the calcium phosphate,
- and conventional purification steps.

5. Process according to one of Claims 1 to 4, **characterised in that** the collagen fibrils have a diameter of 20-500 nm.

6. Process according to one of Claims 1 to 5, **characterised in that** polycarboxylates, such as polyaspartate, polyglutamate, polyphosphoserine or noncollagenic proteins or phosphoproteins are added.

7. Process according to one of Claims 1 to 6, **characterised in that** native collagen is employed.

8. Process for the production of a collagen/calcium phosphate composite starting from mineralised collagen fibrils produced by a process according to Claims 1 to 7, **characterised in that** the mineralised collagen fibrils are embedded in a calcium phosphate cement consisting of calcium phosphates, such as tetracalcium phosphate, calcium hydrogenphosphate, calcium hydroxylapatite, calcium dihydrogenphosphate and/or calcium carbonate, sodium phosphates and/or mixtures thereof, in a collagen:CaP ratio by weight of from 1:200 to 1:1.

9. Process according to Claim 8, **characterised in that** the embedding of the mineralised collagen fibrils is not oriented.

10. Process according to Claim 8, **characterised in that** oriented embedding of the mineralised collagen fibrils takes place through laminates of oriented fibrils.

11. Use of collagen fibrils produced according to one of Claims 1 to 7 for the production of bone replacement material.

## Revendications

1. Procédé de production de fibrilles de collagène minéralisées, **caractérisé en ce que** la formation et la minéralisation de fibrilles ont lieu en une seule étape de procédé.

2. Procédé selon la revendication 1, **caractérisé en ce que** la formation de fibrilles débute avant la minéralisation.

3. Procédé selon les revendications 1 et/ou 2, **caractérisé en ce que** du collagène non réticulé est employé.

4. Procédé selon les revendications 1 et/ou 3, constitué par les étapes réactionnelles :
- de mélange d'une solution acide de collagène comprenant un sel de calcium solubilisé, avec une solution tampon comprenant du phosphate et du chlorure de sodium,
- d'assemblage du collagène en fibrilles par l'incubation d'un précurseur à une température convenable et la minéralisation du phosphate de calcium,
- et des étapes de purification classiques.

5. Procédé selon l'une des revendications 1 à 4, **caractérisé en ce que** les fibrilles de collagène ont un diamètre allant de 20 à 500 nm.

6. Procédé selon l'une des revendications 1 à 5, **caractérisé en ce que** l'on ajoute des polycarboxylates, tels que le polyaspartate, le polyglutamate, la polyphosphosérine ou des protéines non collagéniques ou des phosphoprotéines.

7. Procédé selon l'une des revendications 1 à 6, **caractérisé en ce que** l'on emploie du collagène natif.

8. Procédé de production d'un composite collagène/ phosphate de calcium, à partir des fibrilles de collagène minéralisées produites par un procédé selon les revendications 1 à 7, **caractérisé en ce que** les fibrilles de collagène minéralisées sont incluses dans un ciment de phosphate de calcium constitué par des phosphates de calcium, tels que le phosphate tétracalcique, l'hydrogénophosphate de calcium, l'hydroxyapatite de calcium, le dihydrogénophosphate de calcium et/ou le carbonate de calcium, les phosphates sodiques et/ou des mélanges de ceux-ci, dans un rapport pondéral collagène:PCa allant de 1:200 à 1:1.

9. Procédé selon la revendication 8, **caractérisé en ce que** l'inclusion des fibrilles de collagène minéralisées n'est pas orientée.

10. Procédé selon la revendication 8, **caractérisé en ce que** l'inclusion orientée des fibrilles de collagène minéralisés se produit par l'intermédiaire de feuillets de fibrilles orientées.

11. Utilisation de fibrilles de collagène produites selon l'une des revendications 1 à 7 pour la production de matériau de remplacement d'os.
